# EUROPEAN PATENT APPLICATION

(11) **EP 1 731 154 A1**
(43) Date of publication of application: **13.12.2006**
(21) Application number: 05090167.7
(22) Date of filing: 07.06.2005
(51) Int. Cl.: A61K 31/502, A61K 9/16, A61K 9/20, A61K 9/28, A61P 35/00

(54) **Immediate-release and high-drug-load pharmaceutical formulations of micronised (4-chlorophenyl)|4-(4-pyridylmethyl)pht halazin-1-yl| and salts thereof**

(71) Applicant: Schering Aktiengesellschaft, 13342 Berlin (DE)
(72) Inventor: Backensfeld, Thomas, 13127 Berlin (DE); Wagner, Torsten, 13127 Berlin (DE); Jürgens, Kai Christian, 13347 Berlin (DE); Funke, Adrian, 10587 Berlin (DE)

(57) **Abstract**

The invention relates to immediate-release and high-drug-load solid pharmaceutical formulations comprising micronised (4-chlorophenyl)[4-(4-pyridylmethyl)-phthalazin-1-yl] as well as pharmaceutically acceptable salts thereof.

## Description

### FIELD OF THE INVENTION

The present invention relates to immediate-release and high-drug-load solid pharmaceutical formulations comprising micronised (4-chlorophenyl)[4-(4-pyridylmethyl)-phthalazin-1-yl] as well as pharmaceutically acceptable salts thereof.

### BACKGROUND OF THE INVENTION

Two processes, namely the *de novo* formation of vessels from differentiating endothelial cells or angioblasts in the developing embryo (vasculogenesis) and the growth of new capillary vessels from existing blood vessels (angiogenesis), are involved in the development of the vascular systems of animal organs and tissues. Transient phases of new vessel formation (neovascularisation) also occur in the adult body, for example during the menstrual cycle, during pregnancy or during wound healing.

However, a number of diseases are known to be associated with deregulated angiogenesis, for example retinopathies, psoriasis, haemangioblastoma, haemangioma, and neoplastic diseases (solid tumours). Furthermore, the complex processes of vasculogenesis and angiogenesis have been found to involve a whole range of molecules, especially angiogenic growth factors and their endothelial receptors, as well as cell adhesion molecules.

Recent findings have shown that during embryonic development, during normal growth and in a wide number of pathological conditions and diseases, the angiogenic factor known as "Vascular Endothelial Growth Factor" (VEGF) forms part of the network regulating the growth and differentiation of the vascular system and its components (G. Breier et al., Trends in Cell Biology (1996) 6,454-456 and references cited therein).

VEGF, or more specifically VEGF-A, is a dimeric, disulfide-linked 46 kDa glycoprotein and is structurally related to "Platelet-Derived Growth Factor" (PDGF). It is produced by normal cell lines and tumour cell lines. VEGF is an endothelial cell-specific mitogen and shows angiogenic activity in *in vivo* test systems (e.g. rabbit cornea). VEGF is chemotactic for endothelial cells and monocytes, and induces plasminogen activators in endothelial cells, which are then involved in the proteolytic degradation of the extracellular matrix during formation of capillaries. A number of splice variants of VEGF-A are known which show comparable biological activity, but which differ in the type of cells that secrete them and in their heparin-binding capacity. In addition, there are other members of the VEGF family, such as "Placental Growth Factor" (PLGF), VEGF-B, VEGF-C and VEGF-D.

A large number of human tumours, especially gliomas and carcinomas, express high levels of the VEGF variants and their receptors. This has led to the hypothesis that the VEGF released by tumour cells could stimulate the growth of blood capillaries and the proliferation of tumour endothelium in a paracrine manner and thus, through the improved blood supply, accelerates tumour growth. Increased VEGF expression could explain the occurrence of cerebral oedema in patients with glioma. Direct evidence of the role of VEGF as a tumour angiogenesis factor *in vivo* has been obtained from studies in which VEGF expression or VEGF activity was inhibited. This was achieved with antibodies which inhibit VEGF activity, with dominant-negative VEGFR-2 mutants which inhibited signal transduction, as well as with use of antisense-VEGF RNA techniques. All approaches led to a reduction in the growth of glioma cell lines or other tumour cell lines *in vivo* as a result of inhibited tumour angiogenesis.

There are three VEGFR receptors with different affinities to the ligands. VEGF-A binds to VEGFR1 and VEGFR2; VEGF-B and Placental Growth Factor bind to VEGFR1; VEGF-A and processed forms of VEGF-C and VEGF-D bind to VEGFR-2; VEGF-C and VEGF-D bind to VEGFR-3.

VEGFR-3 is especially important for the growth of lymphatic vessels which play a role in tumour and metastases formation. Also in another diseases state, asthma, the lymphatic tissue is of major importance as it does remain in the alveoli after an acute inflammation and does not resolve like the blood vessels. This leads to the continuing susceptibility to stimulation by foreign agents.

All these receptors are transmembrane proteins. The signal of the VEGF variants is transmitted via the dimerisation of two receptor molecules inducing thereby an activation of the enzymatic activity at the intracellular C-terminal end. The enzymatic activity is a signal kinase, which transfers phosphates from ATP to itself (autophosphorylation) and to downstream signal molecules. This allows for interaction with an entire intracellular signal cascade eventually leading to endothelial cell proliferation and migration. The macroscopic result is the formation of new vessels.

WO 98/35958 describes generically a series of phthalazine derivatives with angiogenesis inhibiting activity and specifically (4-chlorophenyl)[4-(4-pyridylmethyl)-phthalazin-1-yl], including salts thereof, in particular the succinic acid salt thereof, as an interesting candidate for treatment of tumours. This compound is an inhibitor of all three VEGFR kinases. The inhibition is not dependent on a specific ligand, but blocks all the signals.

(4-chlorophenyl)[4-(4-pyridylmethyl)-phthalazin-1-yl] has the chemical structure set forth below:

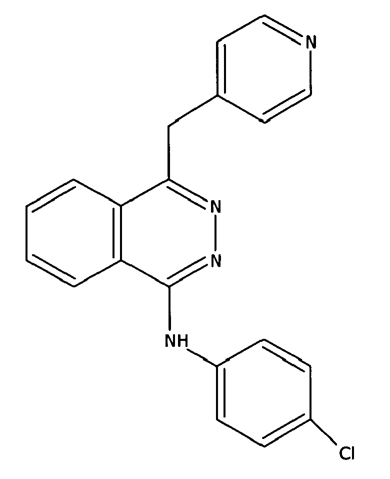

The solubility of (4-chlorophenyl)[4-(4-pyridylmethyl)-phthalazin-1-yl] is extremely dependent on pH. For example, the succinate salt of (4-chlorophenyl)[4-(4-pyridylmethyl)-phthalazin-1-yl] (hereinafter referred to as "pynasunate") has a reasonable solubility at very low pH values, whereas the solubility decreases significantly as the pH is increased:

| pH | Buffered | Solubility (mg/ml) | |
|---|---|---|---|
| | | 37°C | 20°C |
| 1.0 | no | 108 | |
| 1.1 | yes | | 83 |
| 2.0 | no | 146 | |
| 3.0 | yes | 7.9 | |
| 3.1 | yes | | 7.2 |
| 3.6 | no | 0.35 | |
| 3.7 | no | | 0.34 |
| 4.5 | yes | 0.02 | |
| 5.0 | yes | 3.7 x 10⁻³ | 2.9 x 10⁻³ |
| 7.0 | yes | 7.1 x 10⁻⁴ | 3.1 x 10⁻⁴ |

Since the drug substance is absorbed in the small intestine, where the pH is usually above 5, it is of utmost importance that essentially all of the drug substance is dissolved before entering the small intestine, i.e. essentially all of the drug substance should be dissolved in the gastric juice. Evidently, in order to obtain satisfactory absorption of the drug substance, the drug substance must be administered in an immediate-release formulation.

Traditionally, micronisation has been used for the purpose of ensuring adequate release of a drug substance. However, the present inventors surprisingly found that even with a micronised drug substance, a reliable and immediate-release of the drug substance from a solid pharmaceutical formulation was difficult to achieve. For example, as part of the present research program intermediate capsule formulations containing 67 mg pynasunate (21.6% by weight) and 134 mg pynasunate (43.2% by weight) were prepared. It was found, however, that such formulations showed incomplete and highly variable *in vitro* dissolution properties, cf. Comparative Example 1 herein.

Accordingly, there is a need for a robust immediate-release formulation of the drug substance.

It was found that by incorporation of 0.5% surfactant (sodium lauryl sulfate (SDS)) to the inner phase of the granules, the *in vitro* dissolution properties could be improved significantly, cf. Comparative Example 2 herein. However, as will be acknowledged by the skilled person, the use of surfactants/wetting agents in the manufacturing process is preferably avoided. Furthermore, it is known that if using a wet granulation step for manufacturing granules, overwetting has to be completely avoided, and that the amount of granulation liquid is very critical to the granulation process and can only be varied within very limited ranges.

In addition, clinical studies have revealed that about ten of the 134 mg pynasunate capsules (cf. Comparative Example 2 herein) have to be administered to cancer patients per day, in addition to other medicaments, which, in turn, leads to low patient compliance.

It is therefore evident that in addition to the need for a robust immediate-release formulation of the drug substance, there is also a need for a formulation having a high-drug-load.

The present inventors have surprisingly found that a formulation can be prepared which has a high-drug-load, a robust immediate-release profile and which does not necessarily need addition of surfactant. Additionally, the wet granulation step of the process according to the invention was found to be robust against overwetting, i.e. against addition of too much granulation liquid.

Thus, the object of the present invention is therefore to provide a high-load solid pharmaceutical formulation of micronised (4-chlorophenyl)[4-(4-pyridylmethyl)-phthalazin-1-yl], or a pharmaceutically acceptable salts thereof, which exhibits a reproducible immediate-release of the drug substance under the conditions prevailing in the gastric juice.

This object is met by the solid pharmaceutical formulations defined in the appended claims.

### SUMMARY OF THE INVENTION

In a first aspect the present invention relates to a solid pharmaceutical formulation comprising
a) at least 50% by weight of the total formulation of micronised (4-chlorophenyl)[4-(4-pyridylmethyl)-phthalazin-1-yl], or a pharmaceutically acceptable salt thereof; and
b) at least one carboxymethylcellulose-based disintegrant,
wherein at least 70% of said (4-chlorophenyl)[4-(4-pyridylmethyl)-phthalazin-1-yl], or a pharmaceutical acceptable salt thereof, is dissolved from said solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate.

In a further aspect the present invention relates to a solid dosage form, in particular a solid unit dosage form, comprising the solid pharmaceutical formulation of the invention.

In a still further aspect the present invention relates to the solid pharmaceutical formulation of the invention for use as a medicament.

In an even further aspect the present invention relates to the use of the solid pharmaceutical formulation of the invention for the manufacture of a medicament for the treatment of cancer, as well as to a method of treating cancer, said method comprising administering a therapeutically effective amount of the solid pharmaceutical formulation of the invention to a patient in need thereof.

Further aspects of the present invention will be apparent from the below description and the appended claims.

### BRIEF DESCRIPTION OF THE DRAWINGS

Fig. 1 shows the particle size volume distribution of micronised pynasunate used in the Examples.
Fig. 2 shows a typical flow chart for the manufacturing of tablets where the disintegrant is incorporated in the inner phase.
Fig. 3 shows a typical flow chart for the manufacturing of tablets where the disintegrant is incorporated in the outer phase.
Fig. 4 shows the dissolution properties of the tablets prepared according to Examples 1 and 3A-3D where the disintegrant is incorporated in the inner phase. Method: USP 28 Paddle Method; Dissolution media: 0.05 M KH₂PO₄/HCl buffer adjusted to pH 3.0; Temperature: 37°C; Stirring rate: 50 rpm. ◆: Croscarmellose sodium; •: Croscarmellose calcium; △: Maize starch; o: Potato starch; X: Rice starch.
Fig. 5 shows the dissolution properties of the tablets prepared according to Examples 2 and 4A-4E where the disintegrant is incorporated in the outer phase. Method: USP 28 Paddle Method; Dissolution media: 0.05 M KH₂PO₄/HCl buffer adjusted to pH 3.0; Temperature: 37°C; Stirring rate: 50 rpm. ◆: Croscarmellose sodium; •: Croscarmellose calcium; +: Cross-linked povidone; △: Maize starch; o: Potato starch; X: Rice starch.
Fig. 6 shows the dissolution properties of the tablets prepared according to Example 1 and the capsules prepared in Comparative Example 2 (Formulation D). Method: USP 28 Paddle Method; Dissolution media: 0.05 M KH₂PO₄/HCl buffer adjusted to pH 3.0; Temperature: 37°C; Stirring rate: 50 rpm. •: tablet; △: capsule.

### DETAILED DESCRIPTION OF THE INVENTION

As explained above, the present invention provides an immediate-release and high-load solid pharmaceutical formulation comprising the drug substance (4-chlorophenyl)[4-(4-pyridylmethyl)-phthalazin-1-yl], or a pharmaceutically acceptable salt thereof, in micronised form.

### Solid pharmaceutical formulation

When used herein, the term "micronised" is intended to mean that the particle size distribution is so that at least 90% of the particles have a particle diameter of less than 30 µm (calculated from the volume distribution curve under the presumption of spherical particles), i.e. a d₉₀ value of at the most 30 µm. It is well-known to the skilled person that parameters used to describe a given particle size distribution may vary considerably dependent on the specific equipment and settings used. Therefore, it is important to note that whenever the terms "particle size distribution", "particle diameter", "d₅₀", "d₉₀", "d₉₅", "d₉₉", etc. are used herein it should be understood that the specific values or ranges used in connection therewith are always meant to be determined from the volume distribution curve under the presumption of spherical particles using laser diffraction and the conditions set forth in the section entitled "Determination of particle size distribution" herein.

In one embodiment of the invention, the d₉₀ value of (4-chlorophenyl)[4-(4-pyridylmethyl)-phthalazin-1-yl], or a pharmaceutically acceptable salt thereof, is at the most 25 µm, such as at the most 20 µm, e.g. at the most 18 µm, or at the most 16 µm. In a further embodiment of the invention, the d₉₀ value of (4-chlorophenyl)[4-(4-pyridylmethyl)-phthalazin-1-yl], or a pharmaceutically acceptable salt thereof, may be even lower, such as at the most 14 µm, e.g. at the most 12 µm, at the most 10 µm, at the most 8 µm, at the most 6 µm, at the most 5 µm or at the most 4 µm. Analogously, the d₅₀ value of (4-chlorophenyl)[4-(4-pyridylmethyl)-phthalazin-1-yl], or a pharmaceutically acceptable salt thereof, will typically be at the most 10 µm, such as at the most 9 µm, e.g. at the most 8 µm, at the most 7 µm or at the most 6 µm. In a further embodiment of the invention, the d₅₀ value of (4-chlorophenyl)[4-(4-pyridylmethyl)-phthalazin-1-yl], or a pharmaceutically acceptable salt thereof, may be even lower, such as at the most 5 µm, e.g. at the most 4 µm, at the most 3 µm or at the most 2 µm.

Stated differently, the d₉₀ value of (4-chlorophenyl)[4-(4-pyridylmethyl)-phthalazin-1-yl], or a pharmaceutically acceptable salt thereof, is typically in the range from 0.1-30 µm, such as in the range from 0.5-30 µm, e.g. in the range from 0.5-25 µm. In a preferred embodiment of the invention the d₉₀ value of (4-chlorophenyl)[4-(4-pyridylmethyl)-phthalazin-1-yl], or a pharmaceutically acceptable salt thereof, is in the range from 0.5-20 µm, such as in the range from 1-20 µm, e.g. in the range from 2-18 µm, in particular in the range from 4-18 µm. Analogously, the d₅₀ value of (4-chlorophenyl)[4-(4-pyridylmethyl)-phthalazin-1-yl], or a pharmaceutically acceptable salt thereof, will typically be in the range from 0.1-10 µm, such as in the range from 0.5-10 µm, e.g. in the range from 1-8 µm, in the range from 2-8 µm or in the range from 2-6 µm.

As will be understood from the present disclosure, including the Examples provided herein, it is of utmost importance that the drug substance is released in a fast and reliable manner under acidic conditions. Thus, in the present context, the terms "fast-release" or "immediate-release" mean that at least 70% of the drug substance (i.e. (4-chlorophenyl) [4-(4-pyridylmethyl)-phthalazin-1-yl] or a pharmaceutically acceptable salt thereof) is dissolved from the solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate. In a preferred embodiment of the invention at least 75%, more preferably at least 80%, of the drug substance is dissolved from the solid pharmaceutical formulation within 30 minutes as determined by the USP 28 Paddle Method described herein.

As will also be understood from the present disclosure it is of utmost importance that the solid pharmaceutical formulation contains a "high-load" of drug substance due to the relative large amount of drug substance needed for administration to patients. Thus, in the present context, the terms "high-load" or "high-drug-load" mean that the solid pharmaceutical formulation contains at least 50% by weight, calculated on the basis of the total weight of the formulation, of the drug substance (i.e. (4-chlorophenyl) [4-(4-pyridylmethyl)-phthalazin-1-yl] or a pharmaceutically acceptable salt thereof). In an interesting embodiment of the invention the solid pharmaceutical formulation comprises at least 55% by weight, at least 60% by weight, at least 65% by weight, at least 70% by weight, at least 75% by weight, at least 80% by weight, at least 85% by weight or at least 90% by weight of the drug substance, calculated on the basis of the total weight of the formulation. Stated differently, the solid pharmaceutical formulation typically comprises 50-90% by weight, calculated on the basis of the total weight of the formulation, of the drug substance (i.e. (4-chlorophenyl) [4-(4-pyridylmethyl)-phthalazin-1-yl] or a pharmaceutically acceptable salt thereof). In an interesting embodiment of the invention the solid pharmaceutical formulation comprises 50-85% by weight, such as 55-80% by weight, e.g. 60-75% by weight, preferably 60-70% by weight, such as 65-70% by weight of the drug substance, calculated on the basis of the total weight of the formulation.

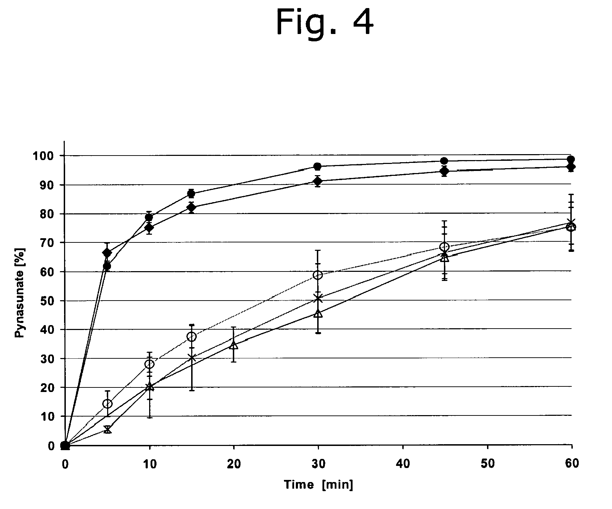

The present invention is, at least in part, based on the surprising discovery that incorporation of a carboxymethylcellulose-based disintegrant in the solid pharmaceutical formulation seems necessary in order to obtain a sufficient fast and reliable release, whereas other commonly used disintegrants proved less useful, cf. Figs. 4-6 herein. The solid pharmaceutical formulation typically comprises 1-25% by weight of the carboxymethylcellulose-based disintegrant, calculated on the basis of the total weight of the formulation. In a preferred embodiment of the invention, the solid pharmaceutical formulation comprises 1-20% by weight of the carboxymethylcellulose-based disintegrant, such as 1-15% by weight of the carboxymethylcellulose-based disintegrant, e.g. 1-10% by weight of the carboxymethylcellulose-based disintegrant, preferably 1-7.5% by weight of the carboxymethylcellulose-based disintegrant, such as 1-5% by weight of the carboxymethylcellulose-based disintegrant, e.g. 2-5% by weight of the carboxymethylcellulose-based disintegrant, more preferably 3-4% by weight of the carboxymethylcellulose-based disintegrant, such as about 3.5% by weight of the carboxymethylcellulose-based disintegrant, calculated on the basis of the total weight of the formulation.

The carboxymethylcellulose-based disintegrant may be present as free acid, but is preferably in the form of a salt, e.g. in the form of an alkali metal salt, such as the potassium salt or the sodium salt, in particular the sodium salt, or in the form of a salt of a divalent metal ion, such as the magnesium salt, the calcium salt or the zinc salt, in particular the calcium salt. The carboxymethylcellulose-based disintegrant may be cross-linked or non-cross-linked. Specific examples of preferred non-cross-linked carboxymethylcellulose-based disintegrant include carboxymetylcellulose calcium (carmellose calcium) and carboxymetylcellulose sodium (carmellose sodium), in particular carboxymetylcellulose calcium. In a highly preferred embodiment of the invention the carboxymethylcellulose-based disintegrant is cross-linked. Furthermore, the carboxymethylcellulose-based disintegrant is preferably in the form of a salt, in particular in the form of the sodium salt (also known as croscarmellose sodium). Croscarmellose sodium is commercially available under the tradenames Ac-Di-Sol®, Explocel® and Solutab®.

The drug substance itself may be the free base, i.e. (4-chlorophenyl)[4-(4-pyridylmethyl)-phthalazin-1-yl] or a pharmaceutically acceptable salt thereof, in particular an acid addition salt. Such salts may be formed from suitable inorganic or organic acids. Examples of suitable inorganic acids include the halogen acids, such as hydrochlorid acid; sulfuric acid; and phosphoric acid. Examples of suitable organic acids include carboxylic, phosphonic, sulfonic or sulfamic acids, such as acetic acid, propionic acid, octanoic acid, decanoic acid, dodecanoic acid, glycolic acid, lactic acid, 2-hydroxybutyric acid, gluconic acid, glucose monocarboxylic acid, fumaric acid, succinic acid, adipic acid, pimelic acid, suberic acid, azelaic acid, malic acid, tartaric acid, citric acid, glucaric acid, galactaric acid; amino acids, such as glutamic acid, aspartic acid, N-methylglycine, acetylaminoacetic acid, N-acetylasparagine and N-acetylcysteine; pyruvic acid, acetoacetic acid, phosphoserine, 2- or 3-glycerophosphoric acid, glucose-6-phosphoric acid, glucose-1-phosphoric acid, fructose-1,6-bis-phosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, mandelic acid, cinnamic acid, glucuronic acid, galacturonic acid, methane- or ethane-sulfonic acid, 2-hydroxyethanesulfonic acid, ethane-1,2-disulfonic acid, 2-, 3-, or 4-methylbenzenesulfonic acid, methylsulfuric acid, ethylsulfuric acid, dodecylsulfuric acid, N-cyclohexylsulfamic acid, N-methyl-, N-ethyl-, or N-propyl-sulfamic acid, or other organic acids, such as ascorbic acid.

In the most preferred embodiment of the invention the drug substance is the succinate salt of (4-chlorophenyl)[4-(4-pyridylmethyl)-phthalazin-1-yl].

Herein, the term "pynasunate" refers to the succinate salt of (4-chlorophenyl)[4-(4-pyridylmethyl)-phthalazin-1-yl], i.e. (4-chlorophenyl)[4-(4-pyridylmethyl)-phthalazin-1-yl]ammonium hydrogen succinate. Pynasunate is described in WO 98/35958.

The solid pharmaceutical formulation of the invention may, in addition to the carboxymethylcellulose-based disintegrant and micronised (4-chlorophenyl)[4-(4-pyridylmethyl)-phthalazin-1-yl], or a pharmaceutically acceptable salt thereof, contain one or more additional pharmaceutically acceptable excipients. These excipients may, for example, be:
- Inert diluents or fillers,
   such as sucrose, sorbitol, sugars, mannitol, microcrystalline cellulose, starches, sodium chloride, sodium phosphate, calcium carbonate, calcium phosphate, calcium sulfate or lactose, e.g. lactose monohydrate. The inert diluent or filler is typically present in an amount from 1-50% by weight of the total formulation. Preferably, the inert diluent or filler is present in an amount from 10-40% by weight of the total formulation, more preferably in an amount from 20-30% by weight of the total formulation. In a preferred embodiment of the invention, the inert filler or diluent is lactose, in particular lactose monohydrate.
- Binders,
   such as sucrose, glucose, sorbitol, acacia, alginic acid, sodium alginate, gelatine, starch, pregelatinised starch, microcrystalline cellulose, magnesium aluminium silicate, carboxymethylcellulose sodium (CMC sodium), methylcellulose, ethylcellulose, hydroxypropylmethylcellulose (HPMC), polyvinylacetate, polyethylene glycol or polyvinylpyrrolidone, e.g. PVP K12, PVP K15, PVP K17, PVP K25, PVP K30, PVP K60, PVP K90, PVP K120 or combinations thereof. The binder is typically present in an amount from 0.5-30% by weight of the total formulation. Preferably, the binder is present in an amount from 1-20% by weight of the total formulation, such as from 1-15% by weight of the total formulation, e.g. from 1-10% by weight of the total formulation, more preferably in an amount from 1-5% by weight of the total formulation. In a preferred embodiment of the invention, the binder is HPMC, in particular HPMC having viscosity grade 3 or 5.
- Lubricants, including glidants and antiadhesives,
   such as magnesium stearate, zinc stearate, stearic acid, silicas, hydrogenated vegetable oils or talc. The lubricant is typically present in an amount from 0.1-10% by weight of the total formulation. Preferably, the lubricant is present in an amount from 0.2-5% by weight of the total formulation, such as from 0.5-5% by weight of the total formulation, e.g. from 1-5% by weight of the total formulation, more preferably in an amount from 1-3% by weight of the total formulation. In a preferred embodiment of the invention, the lubricant is magnesium stearate.
- Disintegrants (in addition to the carboxymethylcellulose-based disintegrant),
   such as cross-linked povidone, sodium starch glycolate, maize starch or potato starch.
- Surfactants and wetting agents,
   such as naturally occurring phosphatides, e.g. lechitin or soybean lechitin; condensation products of ethylene oxide with e.g. a fatty acid, a long chain fatty alcohol, or a partial ester derived from fatty acids and a hexitol or a hexitol anhydride, for example polyoxyethylene stearate, polyoxyethylene sorbitol monooleate, polyoxyethylene sorbitan monooleate, etc.; or salts of long-chain aliphatic phosphates, such as sodium lauryl sulphate.

Examples of other pharmaceutically acceptable excipients which may be incorporated in the solid pharmaceutical formulation of the invention include colorants, flavouring agents, plasticizers, humectants, buffering agents, etc.

The solid pharmaceutical formulations of the invention may also contain further drug substances, such as anti-cancer agents.

In those cases where the pharmaceutical formulation is in the form of a solid dosage form, in particular a solid unit dosage form (e.g. a tablet, sachet or capsule, in particular a tablet), the unit dosage form is adapted for oral administration and may be provided with a coating, such as a film coating, a sugar coating, or the like. Thus, a suitable coating for the solid unit dosage form according to the invention may, for example, be a sugar coating or a film coating based on one or more of the ingredients: Hydroxypropylmethylcellulose (HPMC), methylcellulose, ethylcellulose, hydroxyethylmethylcellulose, hydroxypropylcellulose, carboxymethylcellulose sodium, acrylate polymers (e.g. Eudragit®), polyethylene glycols or polyvinylpyrrolidone. Preferably, the coating is a film coating based on HPMC.

In a highly preferred embodiment of the invention, the solid unit dosage form is in the form of a tablet, preferably a coated tablet, more preferably a film-coated tablet. Concerning the tablet aspect it was particular surprising that tablets could be prepared which not only had a significantly higher drug load compared to a reference capsule formulation but which also permitted the elimination of surfactant/wetting agent.

Accordingly, in a preferred embodiment of the invention the formulation of the invention does not contain a wetting agent and/or a surfactant.

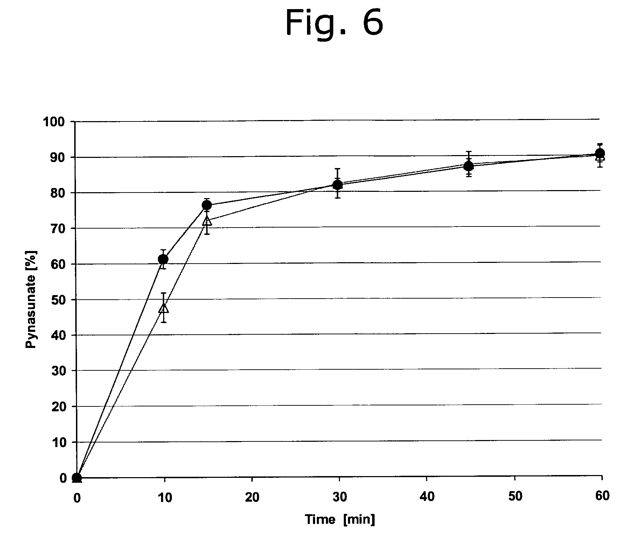

In addition, and as can be seen from Fig. 6 herein, it was surprisingly found that even with almost double drug load, the compressed and film-coated tablets exhibited similar *in vitro* dissolution behaviour as the simple, non-compressed granules contained in capsules. Moreover, as is evident from the *in vivo* data presented in Example 6 herein, the bioavailability of the tablets of the invention was found to be equivalent to the bioavailability of the capsules described in Comparative Example 2 (formulation D) herein.

The uncoated tablet typically has a weight in the range from 400-650 mg, such as in the range of 400-600 mg, e.g. in the range of 425-575 mg, preferably in the range of 450-550 mg, such as in the range of 475-525 mg, e.g. about 500 mg. The coated tablet typically has a weight in the range from 415-665 mg, such as in the range of 415-615 mg, e.g. in the range of 440-590 mg, preferably in the range of 465-565 mg, such as in the range of 490-540 mg, e.g. in the range of 510-520 mg, in particular about 513 mg.

The amount of active drug substance, in particular (4-chlorophenyl)[4-(4-pyridylmethyl)-phthalazin-1-yl]ammonium hydrogen succinate, present in the tablet will typically be in the range from 200-600 mg, preferably 250-500 mg, such as 250-450 mg, e.g. 300-400 mg, more preferably 300-350 mg, such as 325-350 mg, e.g. about 335 mg.

In an interesting embodiment of the invention, the uncoated tablets contain the following ingredients:

| Ingredient | % |
|---|---|
| Drug substance (e.g. pynasunate) | 50-95 |
| Filler (e.g. lactose monohydrate) | 1-50 |
| Binder (e.g. HPMC) | 1-20 |
| Disintegrant (e.g. croscamellose sodium) | 1-25 |
| Lubricant (e.g. magnesium stearate) | 0.1-10 |

The respective uncoated tablets may be coated.

In a preferred embodiment of the invention, the uncoated tablets contain the following ingredients:

| Ingredient | % |
|---|---|
| Drug substance (e.g. pynasunate) | 50-80 |
| Filler (e.g. lactose monohydrate) | 10-40 |
| Binder (e.g. HPMC) | 1-10 |
| Disintegrant (e.g. croscamellose sodium) | 1-10 |
| Lubricant (e.g. magnesium stearate) | 1-5 |

The respective uncoated tablets may be coated.

In an even more preferred embodiment of the invention, the uncoated tablets contain the following ingredients:

| Ingredient | % |
|---|---|
| Drug substance (e.g. pynasunate) | 60-70 |
| Filler (e.g. lactose monohydrate) | 20-30 |
| Binder (e.g. HPMC) | 1-5 |
| Disintegrant (e.g. croscamellose sodium) | 2-5 |
| Lubricant (e.g. magnesium stearate) | 1-3 |

The respective uncoated tablets may be coated.

In the most preferred embodiment of the invention, the uncoated tablets contain the following ingredients:

| Ingredient | % |
|---|---|
| Drug substance (e.g. pynasunate) | 64-69 |
| Filler (e.g. lactose monohydrate) | 22-26 |
| Binder (e.g. HPMC) | 1-3 |
| Disintegrant (e.g. croscamellose sodium) | 3-4 |
| Lubricant (e.g. magnesium stearate) | 1-3 |

The respective uncoated tablets may be coated.

Stated differently, the uncoated tablets may, in a preferred embodiment of the invention, contain the following ingredients:

| Ingredient | Amount (mg) |
|---|---|
| Drug substance (e.g. pynasunate) | 300-400 |
| Filler (e.g. lactose monohydrate) | 50-200 |
| Binder (e.g. HPMC) | 3-25 |
| Disintegrant (e.g. croscamellose sodium) | 5-40 |
| Lubricant (e.g. magnesium stearate) | 2-25 |
| Total | 360-690 |

The respective uncoated tablets may be coated.

In an even more preferred embodiment of the invention, the uncoated tablets contain the following ingredients:

| Ingredient | Amount (mg) |
|---|---|
| Drug substance (e.g. pynasunate) | 300-350 |
| Filler (e.g. lactose monohydrate) | 100-150 |
| Binder (e.g. HPMC) | 5-20 |
| Disintegrant (e.g. croscamellose sodium) | 10-30 |
| Lubricant (e.q. magnesium stearate) | 5-15 |
| Total | 420-565 |

The respective uncoated tablets may be coated.

In the most preferred embodiment of the invention, the uncoated tablets contain the following ingredients:

| Ingredient | Amount (mg) |
|---|---|
| Drug substance (e.g. pynasunate) | 330-340, e.g. 335 |
| Filler (e.g. lactose monohydrate) | 110-130, e.g. 123.5 |
| Binder (e.g. HPMC) | 10-20, e.g. 14 |
| Disintegrant (e.g. croscamellose sodium) | 15-20, e.g. 17.5 |
| Lubricant (e.g. magnesium stearate) | 8-12. e.g. 10 |
| Total | 473-522, e.g. 500 |

The respective uncoated tablets may be coated.

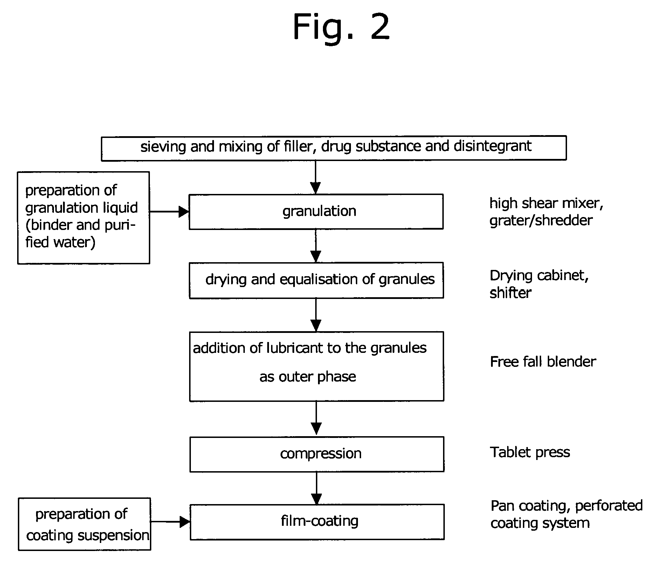

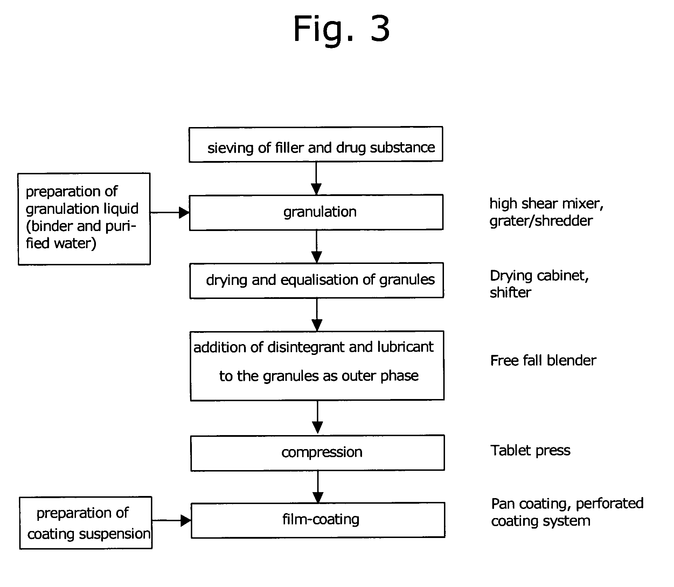

As will be understood from the description *supra* as well as Figs. 2 and 3 herein, the solid unit dosage form of the invention is typically prepared by means of a granulation process, i.e. the micronised drug substance, together with appropriate excipients, is subjected to a granulation process, preferably a wet granulation process, such as a high shear granulation process. After the granulation process, the granules are processed further into the final solid unit dosage form. In one embodiment of the invention the granules may be filled into sachets or capsules, such as hard gelatine capsules. However, in a preferred embodiment of the invention the granules are processed into tablets by compression and subsequently film-coated. As will be understood, the carboxymethylcellulose-based disintegrant may, in one embodiment of the invention, be added before or during the granulation process. In this case the carboxymethylcellulose-based disintegrant can be regarded as an "inner phase" component since it forms part of the granule as such. In another embodiment of the invention, the carboxymethylcellulose-based disintegrant is added to the granules after the granulation process has been completed, i.e. the carboxymethylcellulose-based disintegrant can be regarded as an "outer component". Thus, the carboxymethylcellulose-based disintegrant may be incorporated in the granules of the solid unit dosage form as an "inner phase" component or as an "outer phase" component or as a combination thereof. In a preferred embodiment of the invention, the carboxymethylcellulose-based disintegrant is present as an "outer phase" component.

Accordingly, the present invention is also directed to a process for the preparation of granules comprising a carboxymethylcellulose-based disintegrant (in the outer phase) and at least 50% by weight of the total granule of micronised (4-chlorophenyl)[4-(4-pyridylmethyl)-phthalazin-1-yl], or a pharmaceutically acceptable salt thereof, said process comprising the steps of
i) preparing a liquid medium comprising the binder
ii) preparing a powder mixture comprising at least 50% by weight of the powder mixture of micronized (4-chlorophenyl)[4-(4-pyridylmethyl)-phthalazin-1-yl], or a pharmaceutically acceptable salt thereof
iii) subjecting said powder mixture and said liquid medium to a granulation process to obtain granules;
iv) optionally extruding and shaping the granules
v) optionally drying the granules
vi) adding a carboxymethylcellulose-based disintegrant to the granules
vii) optionally continuing the granulation process
viii) optionally adding a lubricant to the granules
ix) optionally continuing the granulation process and
x) collecting the granules

In a further aspect the present invention is directed to a process for the preparation of granules comprising a carboxymethylcellulose-based disintegrant (in the inner phase) and at least 50% by weight of the total granule of micronised (4-chlorophenyl)[4-(4-pyridylmethyl)-phthalazin-1-yl], or a pharmaceutically acceptable salt thereof, said process comprising the steps of
i) preparing a liquid medium comprising the binder
ii) preparing a powder mixture comprising a carboxymethylcellulose-based disintegrant and at least 50% by weight of the powder mixture of micronized (4-chlorophenyl)[4-(4-pyridylmethyl)-phthalazin-1-yl], or a pharmaceutically acceptable salt thereof,
iii) subjecting said powder mixture and said liquid medium to a granulation process to obtain granules;
iv) optionally extruding and shaping the granules
v) optionally drying the granules
vi) optionally continuing the granulation process
vii) optionally adding a lubricant to the granules
viii) optionally continuing the granulation process and
ix) collecting the granules.

As explained *supra,* the granules obtainable by the processes described above may be further processed into a solid unit dosage form. Accordingly, in a still further aspect the present invention relates to a process for preparing a solid unit dosage form according to the invention, said process comprising the steps of
i) preparing granules according to the methods described herein; and
ii) formulating said granules into solid unit dosage forms.

Furthermore it is well-known to the person skilled in the art that if using a wet granulation step for manufacturing granules, over-wetting has to be avoided completely. For example, the amount of granulating liquid is very critical for the granulation process and can normally only be varied within a very small range Ritschel et al. Die Tablette - Handbuch der Entwicklung, Herstellung und Qualitätssicherung 2. Ed. Editio Cantor Verlag Aulendorf, pp. 270-271). If over-wetting occurs very huge granules will immediately be formed, which are difficult to process further. Accordingly, the entire batch will be lost. Nevertheless, the present inventors have surprisingly found that the manufacturing process of the invention is surprisingly robust with regard to over-wetting. For example, an over-wetted granulated mass can be passed through the shredder and processed to granules with good flowability and the same compression characteristics compared to not-over-wetted batches.

### Medical use

The present invention provides means and methods for treating certain cancers or tumours or tumour angiogenesis, in any suitable animal, preferably in a mammal, and in particular in a human being.

The term "solid malignant tumor" is intended to indicate an abnormal malignant mass of tissue that is not inflammatory, which arises without obvious cause from cells of preexistent tissue, which possesses no physiologic function and which has the ability to invade normal cells and spread throughout the body. Examples of typical solid malignant tumours include breast carcinoma, non-small cell lung cancer, colon carcinoma, renal cell carcinoma and malignant melanoma.

The term "carcinoma" is intended to indicate a malignant tumour of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term "sarcoma" is intended to indicate a malignant tumour growing from connective tissue, such as cartilage, fat, muscles, tendons and bones.

The term "leukaemia" is intended to indicate a blood cancer, i.e. a cancer that originates from the bone marrow and which keeps the marrow from producing normal red and white blood cells and platelets.

The term "lymphoma" refers to a cancer that originates in the nodes or glands of the lymphatic system. The term "glioma", when used herein, is intended to cover a malignant tumor originating from glial cells.

The term "inhibition" or "inhibit" as used in connection with treatment of solid tumours is intended to cover the delayed appearance of primary or secondary tumours, slowed development of primary or secondary tumours, decreased occurrence of primary or secondary tumours, slowed or decreased severity of secondary effects of disease, arrested tumour growth and regression of tumours. The term "reduction" or "reducing" in connection with tumour size is covered by the term "inhibition" or "inhibit". The reduction of the solid tumour refers to a reduction of the tumour volume. For example, a 10% reduction of a solid tumour means that the volume of the treated tumour has been reduced with 10%.

An important aspect of the present invention lies in the therapeutic application of the solid pharmaceutical formulation of the invention.

Thus, the present invention is also directed to a solid pharmaceutical formulation of the invention for use as a medicament. In particular, the present invention is directed to use of the solid pharmaceutical formulation of the invention for the manufacture of a medicament for treatment of cancer. Alternatively stated, the present invention is directed to a method of treating cancer, said method comprising administering a therapeutically effective amount of the solid pharmaceutical formulation of the invention to a patient in need thereof.

In the present context the term "treatment of a cancer" or "treating a mammal having a cancer" is intended to mean that the solid pharmaceutical formulation described herein is administered in a therapeutically effective amount which is sufficient to *i*) inhibit growth of the tumour, *ii)* facilitate tumour regression, i.e. reduce the size of the tumour, *iii)* remove the tumour and/or iv) inhibit cancer cell metastasis.

The solid pharmaceutical formulation will be administered to patients in a "therapeutically effective" dose, i.e. in a dose that is sufficient to produce the desired effects in relation to the condition for which it is administered. The exact dose will depend on the cancer form to be treated, and will be ascertainable by one skilled in the art using known techniques. A suitable dose of the drug substance, in particular pynasunate, is contemplated to be in the range of about 0.5-3 g/patient/day, preferably 1-2 g/patient/day, such as 1-1.5 g/patient/day, e.g. 1.3-1.4 g/patient/day, in particular 1.34 g/patient/day.

In a very interesting embodiment of the invention said cancer is a carcinoma, such as a carcinoma selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumours, in particular selected from the group consisting of malignant melanoma, non-small cell lung cancer, breast carcinoma, colon carcinoma and renal cell carcinoma.

Thus, in one embodiment of the invention said cancer is malignant melanoma, such as superficial spreading melanoma, nodular melanoma, lentigo maligna melanoma, acral melagnoma, amelanotic melanoma or desmoplastic melanoma. In another embodiment of the invention said cancer is non-small cell lung cancer. In still another embodiment of the invention said cancer is breast carcinoma. In a further embodiment of the invention said cancer is colon carcinoma. In an even further embodiment of the invention said cancer is renal cell carcinoma.

In a further interesting embodiment of the invention said cancer is a sarcoma, such as a sarcoma selected from the group consisting of osteosarcoma, Ewing's sarcoma, chondrosarcoma, malignant fibrous histiocytoma, fibrosarcoma and Kaposi's sarcoma.

In an even further interesting embodiment of the invention said cancer is a glioma.

As will be understood by the skilled person, the above-mentioned cancer types (i.e. carcinomas, sarcomas and gliomas) are characterised by the presence of solid tumours.

In an even further interesting embodiment of the invention said cancer is a lymphoma, such as a lymphoma selected from the group consisting of Hodgkin's disease, non-Hodgkin's disease, B-cell lymphoma, T-cell lymphoma, follicular lymphoma, Burkitt's lymphoma and mycosis fungoides.

In still another interesting embodiment of the invention said cancer is a myeloma, such as multiple myeloma.

One important use in cancer therapy is the reduction and blockade of ascites formation in abdominal tumours of different origin (e.g. uterine endometrium, ovarial cancer, colon cancer) also for mesothelioma.

In addition to its use as anticancer agent the formulation of the invention can be used for other diseases characterised by overshooting angiogenesis, like macular degeneration due to vessel in-growth, corneal transplatation due to lymphatic vessel ingrowth and thereby breakage of the immune privilege. It is helpful in diseases like rheumatoid arthritis with vessels growing ectopically towards the joints. As mentioned above it is effective in asthma. Also diseases with female cycle associated vessel growth are influenced beneficially as is the case for endometriosis.

It will be understood that an even more effective treatment of the various cancer forms may be obtained by combination therapy where the solid pharmaceutical formulation of the invention is combined with a suitable chemotherapeutic agent and/or is combined with radiotherapy and/or is combined with surgery.

Thus, a further aspect the present invention relates to the use of a solid pharmaceutical formulation of the invention for the manufacture of a medicament for treatment of cancer in combination with a chemotherapeutic agent. Analogously, a further aspect of the present invention relates to a method of treating cancer, said method comprising administering a therapeutically effective amount of the solid pharmaceutical formulation of the invention and a chemotherapeutic agent to a patient in need thereof.

Specific examples of suitable chemotherapeutic agents include chemotherapeutic agents selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine).

Other chemotherapeutic agents, which may be useful for the purposes described herein include the chemotherapeutic agents mentioned in column 12, line 62 to column 13, line 42 of US 6,482,802. For a description of these and other chemotherapeutic agents, see The Merck Index, 12th edition, pp. THER 13-14.

It will be understood that the chemotherapeutic agent is selected under due consideration of the actual cancer form. In a preferred embodiment of the invention the following chemotherapeutic agents are used (together with the solid pharmaceutical formulation described herein) for treatment of the following specific cancer forms: malignant melanoma and cisplatin; malignant melanoma and IL-2; renal cell carcinoma and doxorubicin; renal cell carcinoma and IL-2; breast carcinoma and doxorubicin; breast carcinoma and taxol; colon carcinoma and 5-fluorouracil; colon carcinoma and cisplatin; and non-small cell lung cancer and cisplatin.

The invention is further described in the following examples. The examples should not, in any manner, be understood as limiting the generality of the present specification and claims.

### MATERIALS AND METHODS

### Determination of particle size distribution

Determination of the particle size volume distribution of pynasunate was performed by laser diffraction using the following equipment and settings:
- Apparatus:: Sympatec Helos (H0583)
- Dispersion system:: Aerial dry dispersion using Sympatec Rodos module
- Focal length:: 125 mm
- Volume of air stream:: 2 m³/h
- Prepressure:: 2 bar
- Dispersion pressure:: 3 bar
- Sample feed:: Via rotating round brush
- Lens:: R3 (0.9-175 µm)
- Optical concentration:: 0.8 - 20%
- Measurement time:: 2 seconds
- Optical model:: Fraunhofer under the presumption of spherical particles.

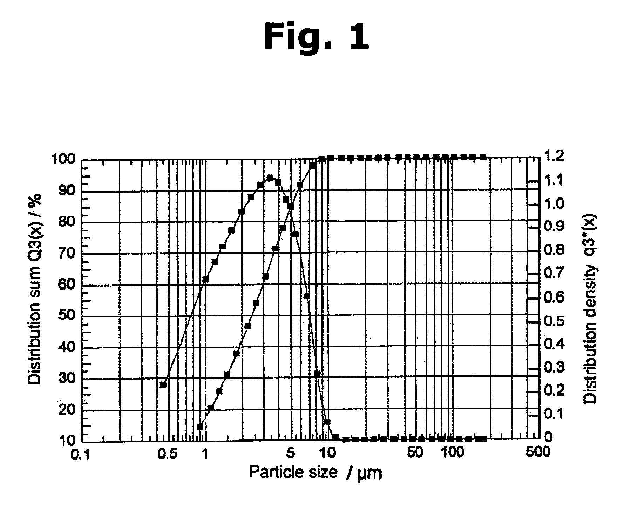

Using the above-indicated settings, the particle size volume distribution of the micronised pynasunate particles used in the Examples herein was so that 90% of the particles had a particle size diameter equal to or less than 15 µm (d₉₀=15 µm), and 50% of the particles had a particle size diameter equal to or less than 6 µm (d₅₀=6 µm). The particle size distribution of the micronised pynasunate particles is shown in Fig. 1.

### Dissolution method

Determination of pynasunate dissolution from film-coated tablets or granules filled into gelatine capsules was performed in accordance with the USP 28 Paddle Method using the following test parameters:
- Apparatus:: USP dissolution apparatus 2 with six covered glass vessels and paddle stirrers
- Medium:: 0.05 M phosphate buffer (KH₂PO₄/HCl), degassed. The pH value was finally adjusted to 3.00±0.05
- Filling volume:: 1000 ml
- Temperature:: 37°C±0.5°C
- Stirring rate:: 50 rpm±2 rpm
- Sampling times:: 5, 10, 15, 30, 45 and 60 minutes
- Detection:: UV-measurement at 316 nm

### EXAMPLES

### Comparative Example 1: Pynasunate capsules

Capsules of the following composition were prepared:

| | Formulation A | | Formulation B | |
|---|---|---|---|---|
| Ingredient | Amount (mg) | % | Amount (mg) | % |
| *Inner phase* | | | | |
| Micronised pynasunate | 67.0 | 21.6 | 134.0 | 43.2 |
| Lactose monohydrate | 161.2 | 52.0 | 103.3 | 33.3 |
| Microcrystalline cellulose | 33.9 | 10.9 | 24.8 | 8.0 |
| Hydroxypropylmethylcellulose (HPMC) *Outer phase* | 13.4 | 4.3 | 13.4 | 4.3 |
| Croscamellose sodium | 31.0 | 10.0 | 31.0 | 10.0 |
| Collodial silicon dioxide | 1.0 | 0.3 | 1.0 | 0.3 |
| Magnesium stearate | 2.4 | 0.8 | 2.4 | 0.8 |
| Total | 310.0 | | 310.0 | |
| Hard gelatine capsule size | 0 | | 0 | |

The dissolution properties of the capsules were investigated using the USP 28 Paddle Method described above. It was found that the dissolution properties of both Formulation A and B were unsatisfactory (less than 80% dissolved after 60 minutes). Furthermore, the obtained data were highly variable.

### Comparative Example 2: Pynasunate capsules containing surfactant

Capsules of the following composition were prepared:

| | Formulation C | | Formulation D | |
|---|---|---|---|---|
| Ingredient | Amount (mg) % | | Amount (mg) % | |
| *Inner phase* | | | | |
| Micronised pynasunate | 67.0 | 19.7 | 134.0 | 39.4 |
| Lactose monohydrate | 187.0 | 55.0 | 120.0 | 35.3 |
| Microcrystalline cellulose | 37.4 | 11.0 | 37.4 | 11.0 |
| Hydroxypropylmethylcellulose (HPMC) | 8.5 | 2.5 | 8.5 | 2.5 |
| Sodium lauryl sulfate (SDS) *Outer phase* | 1.7 | 0.5 | 1.7 | 0.5 |
| Croscamellose sodium | 34.0 | 10.0 | 34.0 | 10.0 |
| Collodial silicon dioxide | 1.7 | 0.5 | 1.7 | 0.5 |
| Magnesium stearate | 2.7 | 0.8 | 2.7 | 0.8 |
| Total | 340.0 | | 340.0 | |
| Hard gelatine capsule size | 0 | | 0 | |

The dissolution properties of the capsules were investigated using the USP 28 Paddle Method described above. It was found that the dissolution properties of both Formulation C and D were satisfactory (more than 70% dissolved after 30 minutes).

### Example 1: High-load pynasunate tablets - disintegrant in inner phase

Tablets of the following composition were prepared:

| | Formulation 1 | |
|---|---|---|
| Ingredient | Amount (mg) | % |
| *Inner phase* | | |
| Micronised pynasunate | 335.0 | 67.0 |
| Lactose monohydrate | 123.5 | 24.7 |
| Hydroxypropylmethylcellulose (HPMC) | 14.0 | 2.8 |
| Croscamellose sodium *Outer phase* | 17.5 | 3.5 |
| Magnesium stearate | 10.0 | 2.0 |
| Total | 500.0 | |

| Coating | | |
|---|---|---|
| Hydroxypropylmethylcellulose (HPMC) | 5.6 | |
| Talc | 1.5 | |
| Titanium dioxide | 4.1 | |
| Ferric oxide pigment, yellow | 1.8 | |
| Film coating weight | 13.0 | |

The tablets were obtained by the granulation/compression process shown in Fig. 2. In short, the tablets were prepared as follows:

8.4 g HPMC was dissolved in 57.0 g purified water. The suspension was stirred until a clear binder solution was achieved. The completeness of the dissolution was visually checked.

134.0 g micronised pynasunate was charged in a high shear mixer with 49.4 g lactose monohydrate. 7.0 g croscarmellose sodium was added. The mixture was blended for 10 min at 200 UpM. 43.6 g of the above-mentioned binder solution was slowly added to the dry blend. The wet mixture was kneaded for 5 min and passed through a grater/shredder. The wet granules were dried in a drying cabinet at 50°C until the relative humidity of the granules achieved 30-55 % relative humidity at room temp. The granules were equalised using a 1.0 mm sieve.

A free fall blender was charged with the granules and 4.0 g magnesium stearate. The mixture was blended for 30 sec at a rotation speed of 20-25 UpM.

Using a single punch press capsule-shaped tablets were manufactured.

### Example 2: High-load pynasunate tablets - disintegrant in outer phase

Tablets of the following composition were prepared:

| | Formulation 2 | |
|---|---|---|
| Ingredient | Amount (mg) | % |
| *Inner phase* | | |
| Micronised pynasunate | 335.0 | 67.0 |
| Lactose monohydrate | 123.5 | 24.7 |
| Hydroxypropylmethylcellulose (HPMC) *Outer phase* | 14.0 | 2.8 |
| Croscamellose sodium | 17.5 | 3.5 |
| Magnesium stearate | 10.0 | 2.0 |
| Total | 500.0 | |

| | | |
|---|---|---|
| Coating | | |
| Hydroxypropylmethylcellulose (HPMC) | 5.6 | |
| Talc | 1.5 | |
| Titanium dioxide | 4.1 | |
| Ferric oxide pigment, yellow | 1.8 | |
| Film coating weight | 13.0 | |

The tablets were obtained by the granulation/compression process shown in Fig. 3. In short, the tablets were prepared as follows:

112.0 g HPMC was dissolved in purified water at a temperature of 50-60°C. The suspension was stirred until a clear binder solution was achieved. The completeness of the dissolution was visually checked.

988.0 g lactose monohydrate was charged in a high shear mixer with 2680.0 g micronised pynasunate. The mixture was blended for 5 min at 80 UpM. 872.0 g of the above-mentioned binder solution was slowly added to the dry blend. The wet mixture was kneaded at a rotation speed of 80 UpM twice for 3-5 min, and the consistence of the granules was visually checked. The wet mass was then passed through a shredder equipped with a 3.0 mm sieve. The wet granules were transferred to a fluid bed dryer and dried with a inlet temperature of 50°C and a air flow of approximately 150 m³/h until a relative humidity of 40-60% was been achieved. The granules were equalised using a 1.0 mm sieve.

A free fall blender was charged with the granules and 140.0 g croscamellose sodium. The mixture was blended for 15 min at a rotation speed of 20-36 UpM. The free fall blender was then charged with 80.0 g magnesium stearate. The mixture was blended for 1 min at a rotation speed of 20-36 UpM.

Using a single punch press capsule-shaped tablets (17.6 x 6.7 mm) were manufactured.

### Example 3A: High-load pynasunate tablets containing CMC calcium in inner phase

Tablets were prepared as described in Example 1, the only difference being that the disintegrant, croscarmellose sodium, was replaced by another disintegrant, namely carmellose calcium.

### Example 3B: High-load pynasunate tablets containing maize starch in inner phase

Tablets were prepared as described in Example 1, the only difference being that the disintegrant, croscarmellose sodium, was replaced by another disintegrant, namely maize starch.

### Example 3C: High-load pynasunate tablets containing potato starch in inner phase

Tablets were prepared as described in Example 1, the only difference being that the disintegrant, croscarmellose sodium, was replaced by another disintegrant, namely potato starch.

### Example 3D: High-load pynasunate tablets containing rice starch in inner phase

Tablets were prepared as described in Example 1, the only difference being that the disintegrant, croscarmellose sodium, was replaced by another disintegrant, namely rice starch.

### Example 4A: High-load pynasunate tablets containing CMC calcium in outer phase

Tablets were prepared as described in Example 2, the only difference being that the disintegrant, croscarmellose sodium, was replaced by another disintegrant, namely carmellose calcium.

### Example 4B: High-load pynasunate tablets containing cross-linked povidone in outer phase

Tablets were prepared as described in Example 2, the only difference being that the disintegrant, croscarmellose sodium, was replaced by another disintegrant, namely cross-linked povidone.

### Example 4C: High-load pynasunate tablets containing maize starch in outer phase

Tablets were prepared as described in Example 2, the only difference being that the disintegrant, croscarmellose sodium, was replaced by another disintegrant, namely maize starch.

### Example 4D: High-load pynasunate tablets containing potato starch in outer phase

Tablets were prepared as described in Example 2, the only difference being that the disintegrant, croscarmellose sodium, was replaced by another disintegrant, namely potato starch.

### Example 4E: High-load pynasunate tablets containing rice starch in outer phase

Tablets were prepared as described in Example 2, the only difference being that the disintegrant, croscarmellose sodium, was replaced by another disintegrant, namely rice starch.

### Example 5: Dissolution

The dissolution properties of the tablets from Examples 1 and 3 (disintegrant in inner phase) were compared using the USP 28 Paddle Method described above. The obtained results are shown in Fig. 4. As can be seen, only tablets containing the disintegrant croscarmellose sodium exhibited sufficient immediate-release of the pynasunate, whereas the release properties of tablets containing maize starch were unsatisfactory.

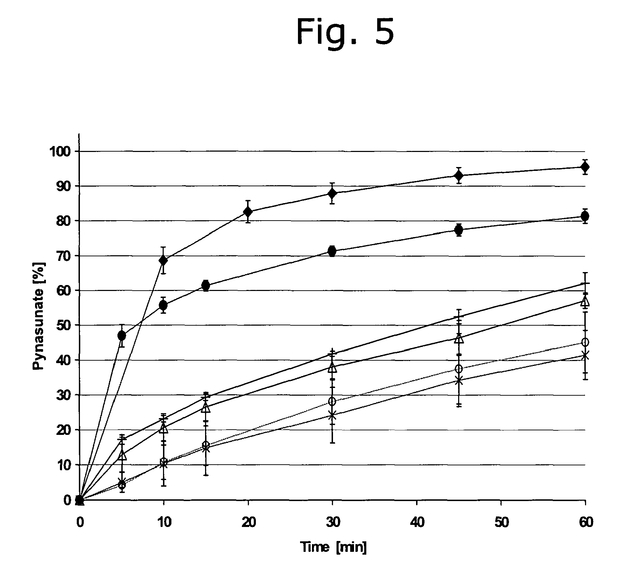

The dissolution properties of the tablets from Examples 2, 4 and 5 (disintegrant in outer phase) were compared using the USP 28 Paddle Method described above. The obtained results are shown in Fig. 5. As can be seen, only tablets containing the disintegrant croscarmellose sodium exhibited sufficient immediate-release of the pynasunate, whereas the release properties of tablets containing maize starch and cross-lined povidone were unsatisfactory.

Finally, the dissolution properties of the Formulation D capsules prepared in Comparative Example 2 were compared to the dissolution properties of the tablets prepared in Example
1. The obtained results are shown in Fig. 6. No difference in the dissolution properties between the tablets and the capsules could be seen.

### Example 6: Bioavailability

A comparison of the *in vivo* bioavailability for the Formulation D capsules prepared in Comparative Example 2 and the tablets prepared in Example 1 was performed. The obtained data are compiled in the below Table:

| Parameter | | Capsule (geometric mean; arithmetic mean) | Tablet (geometric mean; arithmetic mean) |
|---|---|---|---|
| AUC_{0 - τ} | [ng x h/ml] | 21120; | 24201; |
| | | 24921 | 27836 |
| Cₘₐₓ | [ng/ml] | 4768; | 5323; |
| | | 6194 | 6409 |
| t_{½} | [h] | 4.1; | 3.9; |
| | | 4.4 | 4.4 |

## Claims

1. A solid pharmaceutical formulation comprising
a) at least 50% by weight of the total formulation of micronised (4-chlorophenyl)[4-(4-pyridylmethyl)-phthalazin-1-yl], or a pharmaceutically acceptable salt thereof; and
b) at least one carboxymethylcellulose-based disintegrant,
wherein at least 70% of said (4-chlorophenyl)[4-(4-pyridylmethyl)-phthalazin-1-yl], or a pharmaceutical acceptable salt thereof, is dissolved from said solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate.

2. The formulation according to claim 1, wherein at least 75%, preferably at least 80%, of said micronised (4-chlorophenyl)[4-(4-pyridylmethyl)-phthalazin-1-yl], or a pharmaceutically acceptable salt thereof, is dissolved from said solid pharmaceutical formulation within 30 minutes.

3. The formulation according to claim 1 or 2, wherein said carboxymethylcellulose-based disintegrant is cross-linked.

4. The formulation according to any of claims 1-3, wherein said carboxymethylcellulose-based disintegrant is in the form of a salt.

5. The formulation according to claim 4, wherein said salt is the sodium salt.

6. The formulation according to claim 5, wherein said carboxymethylcellulose-based disintegrant is croscarmellose sodium.

7. The formulation according to any of the preceding claims, wherein said micronised (4-chlorophenyl)[4-(4-pyridylmethyl)-phthalazin-1-yl], or a pharmaceutically acceptable salt thereof, has a d₉₀ value of at the most 25 µm, such as at the most 20 µm, e.g. at the most 18 µm, at the most 16 µm, at the most 14 µm, at the most 12 µm, at the most 10 µm, at the most 8 µm, at the most 6 µm, at the most 5 µm or at the most 4 µm when determined as described herein.

8. The formulation according to any of the preceding claims, wherein said micronised (4-chlorophenyl)[4-(4-pyridylmethyl)-phthalazin-1-yl], or a pharmaceutically acceptable salt thereof, has a d₉₀ value is in the range from 0.1-30 µm, such as in the range from 0.5-30 µm, e.g. in the range from 0.5-25 µm, preferably in the range from 0.5-20 µm, such as in the range from 1-20 µm, e.g. in the range from 2-18 µm, in particular in the range from 4-18 µm when determined as described herein.

9. The formulation according to any of the preceding claims, wherein said micronised (4-chlorophenyl)[4-(4-pyridylmethyl)-phthalazin-1-yl], or a pharmaceutically acceptable salt thereof, has a d₅₀ value of at the most 10 µm, such as at the most 9 µm, e.g. at the most 8 µm, at the most 7 µm, at the most 6 µm, at the most 5 µm, at the most 4 µm, at the most 3 µm or at the most 2 µm when determined as described herein.

10. The formulation according to any of the preceding claims, wherein said micronised (4-chlorophenyl)[4-(4-pyridylmethyl)-phthalazin-1-yl], or a pharmaceutically acceptable salt thereof, has a d₅₀ value is in the range from 0.1-10 µm, such as in the range from 0.5-10 µm, e.g. in the range from 1-8 µm, in the range from 2-8 µm or in the range from 2-6 µm when determined as described herein.

11. The formulation according to any of the preceding claims, comprising at least 55% by weight of the total formulation of micronised (4-chlorophenyl)[4-(4-pyridylmethyl)-phthalazin-1-yl], or a pharmaceutically acceptable salt thereof, such as at least 60% by weight, e.g. at least 65% by weight, at least 70% by weight, at least 75% by weight, at least 80% by weight, at least 85% by weight or at least 90% by weight of the total formulation of micronised (4-chlorophenyl)[4-(4-pyridylmethyl)-phthalazin-1-yl], or a pharmaceutically acceptable salt thereof.

12. The formulation according to any of the preceding claims, wherein said formulation comprises 1-25% by weight of the total formulation of the carboxymethylcellulose-based disintegrant, such as 1-20% by weight, e.g. 1-15% by weight, preferably 1-10% by weight, such as 1-7.5% by weight, e.g. 1-5% by weight, more preferably 2-5% by weight, such as 3-4% by weight, e.g. about 3.5% by weight of the total formulation of the carboxymethylcellulose-based disintegrant.

13. The formulation according to any of the preceding claims comprising at least one further pharmaceutically acceptable excipient.

14. The formulation according to any of the preceding claims, wherein said (4-chlorophenyl)[4-(4-pyridylmethyl)-phthalazin-1-yl] is in the form of a pharmaceutically acceptable salt thereof.

15. The formulation according to claim 14, wherein said pharmaceutically acceptable salt thereof is an acid addition salt.

16. The formulation according to claim 15, wherein said pharmaceutically acceptable salt of (4-chlorophenyl)[4-(4-pyridylmethyl)-phthalazin-1-yl] is (4-chlorophenyl)[4-(4-pyridylmethyl)-phthalazin-1-yl]ammonium hydrogen succinate.

17. A solid dosage form comprising a solid pharmaceutical formulation according to any of the preceding claims.

18. A solid dosage form according to claim 17, which is in a unit dosage form.

19. The solid unit dosage form according to claim 18, wherein said solid unit dosage form is adapted for oral administration.

20. The solid unit dosage form according to claim 19, wherein said solid unit dosage form is in the form of a tablet, sachet or capsule.

21. The solid unit dosage form according to claim 20, wherein said solid unit dosage form is in the form of a tablet

22. The tablet according to claim 21, wherein said tablet is coated.

23. The tablet according to claim 22, wherein said tablet is film-coated.

24. A process for the preparation of granules comprising a carboxymethylcellulose-based disintegrant and at least 50% by weight of the total granule of micronised (4-chlorophenyl)[4-(4-pyridylmethyl)-phthalazin-1-yl], or a pharmaceutically acceptable salt thereof, said process comprising the steps of
i) preparing a liquid medium comprising the binder
ii) preparing a powder mixture comprising at least 50% by weight of the powder mixture of micronized (4-chlorophenyl)[4-(4-pyridylmethyl)-phthalazin-1-yl], or a pharmaceutically acceptable salt thereof
iii) subjecting said powder mixture and said liquid medium to a granulation process to obtain granules;
iv) optionally extruding and shaping the granules
v) optionally drying the granules
vi) adding a carboxymethylcellulose-based disintegrant to the granules
vii) optionally continuing the granulation process
viii) optionally adding a lubricant to the granules
ix) optionally continuing the granulation process and
x) collecting the granules

25. A process for the preparation of granules comprising a carboxymethylcellulose-based disintegrant and at least 50% by weight of the total granule of micronised (4-chlorophenyl)[4-(4-pyridylmethyl)-phthalazin-1-yl], or a pharmaceutically acceptable salt thereof, said process comprising the steps of
i) preparing a liquid medium comprising the binder
ii) preparing a powder mixture comprising a carboxymethylcellulose-based disintegrant and at least 50% by weight of the powder mixture of micronized (4-chlorophenyl)[4-(4-pyridylmethyl)-phthalazin-1-yl], or a pharmaceutically acceptable salt thereof,
iii) subjecting said powder mixture and said liquid medium to a granulation process to obtain granules;
iv) optionally extruding and shaping the granules
v) optionally drying the granules
vi) optionally continuing the granulation process
vii) optionally adding a lubricant to the granules
viii) optionally continuing the granulation process and
ix) collecting the granules.

26. Granules obtainable by the process according to claim 24 or 25.

27. A process for preparing a solid unit dosage form according to claim 17, said process comprising the steps of
i) preparing granules according to claim 24 or 25; and
ii) formulating said granules into solid unit dosage forms.

28. The process according to claim 27, wherein said step of formulating said granules into solid dosage forms comprises compressing said granules into tablets.

29. The process according to claim 28, wherein said process further comprises the step of coating said tablets.

30. The process according to claim 27, wherein said step of formulating said granules into solid dosage forms comprises filling the granules into sachets or capsules.

31. A solid unit dosage form obtainable by the process according to any of claims 27-30.

32. A formulation according to any of claims 1-16 or a dosage form according to any of claims 17-23 or 31 for use as a medicament.

33. Use of a formulation according to any of claims 1-16 or a dosage form according to any of claims 17-23 or 31 for the manufacture of a medicament for the treatment of cancer.

34. Use of a formulation according to any of claims 1-16 or a dosage form according to any of claims 17-23 or 31 for the manufacture of a medicament for the treatment of cancer in combination with a chemotherapeutic agent.

35. A method of treating cancer, said method comprising administering a therapeutically effective amount of the formulation according to any of claims 1-16 or a dosage form according to any of claims 17-23 or 31 to a patient in need thereof.

36. A method of treating cancer, said method comprising administering a therapeutically effective amount of the formulation according to any of claims 1-16 or a dosage form according to any of claims 17-23 or 31 and a chemotherapeutic agent to a patient in need thereof.
